# EUROPEAN PATENT APPLICATION

(11) **EP 4 509 150 A1**
(43) Date of publication of application: **19.02.2025**
(21) Application number: 23895526.4
(22) Date of filing: 29.06.2023
(51) Int. Cl.: A61M 5/178, A61M 5/31, A61M 5/315

(54) **DRUG DELIVERY DEVICE**

(71) Applicant: Zhejiang Summed Medtech Co., Ltd., Huzhou, Zhejiang 313000 (CN)
(72) Inventor: GAO, Pengtao, Jiaxing, Zhejiang 314199 (CN); WENG, Jiansen, Taoyuan City, Taiwan (TW)
(74) Representative: Schwerbrock, Florian
(86) International application number: PCT/CN2023/103947
(87) International publication number: WO 2025/000360

(57) **Abstract**

The present invention provides a drug delivery device, which includes a pen cap, a right side of the pen cap is fixedly provided with a drug delivery mechanism, a right end surface of the drug delivery mechanism is provided with a metering adjustment mechanism, the drug delivery mechanism includes a drug administration element and a connection element, in which one side of the drug administration element is connected to one side of the connection element, the connection element includes a pen holder, in which a left side of the pen holder is connected to a right side of the pen cap and is passed through, the metering adjustment mechanism includes an adjustment element and an observation element, in which one side of the adjustment element is connected to one side of the observation element, the adjustment element includes a knob, a left end surface of the knob is connected to a right side of the pen holder. The drug delivery device of the present invention is provided with the metering adjustment mechanism, and is provided with the knob and a scale socket to operate together, so that reciprocation can be performed when adjusting the dosage to be delivered, thereby avoiding waste of medicine, and the button does not move during the adjustment process, thereby the injection can be convenient, the transmission mechanism of the product can be simplified, and the elements can be optimized.

## Description

### FIELD OF THE INVENTION

The present invention relates to a technical field of drug delivery, particularly relates to a drug delivery device.

### BACKGROUND OF THE INVENTION

An injection pen, also known as a medication pen, is a device used to inject medications under the skin. Designed to make injectable medications easier and more convenient to use, so as to improve the patient compliance.

China publication application discloses a drug injection pen with the publication number CN204337431U, which includes a housing and a dosage setting knob that can rotate relative to the housing. The brake assembly is disposed in the housing and has a ratchet component. The driver includes at least one external tooth.

However, there are still includes shortcomings as follows: although the drug injection pen provides a simple assembly that can easily customized by relying on the drug injection device of the abovementioned prior art, the prior art is to arrange the ratchet at the position of the knob, so the knob disclosed in the existing patent only rotates in one direction, resulting in the drug injection device only increase the dose but cannot reduce the dose. If the adjusted dose exceeds the predetermined volume, it is an irreversible and the drug can only be given out through an empty needle, resulting in a waste of the drug.

Accordingly, the present invention designs a drug delivery device to solve the above long-standing technical problems in the prior art.

### SUMMARY OF THE INVENTION

The object of the present invention is to provide a drug delivery device to solve the technical problem raised in above prior art.

In order to achieve the above object, the present invention provides the following technical solution: a drug delivery device includes a pen cap, a right side of the pen cap is provided with a drug delivery mechanism, a right end surface of the drug delivery mechanism is provided with a metering adjustment mechanism, an inner wall of a right end surface of the pen cap is provided with a ratchet socket, and an inner wall of the ratchet socket is provided with a driving ratchet;
the drug delivery mechanism further includes a drug administration element and a connection element, one side of the drug administration element is connected to one side of the connection element;
the connection element includes a pen holder, a right side of the pen holder is connected through the ratchet socket, a driving rod is provided inside the pen holder, a pushing rod is provided inside the driving rod, one side of the driving rod is slidingly connected to an inner wall of the driving rod, and one side of the pushing rod is connected to one side of the driving ratchet;
the metering adjustment mechanism further includes an adjustment element and an observation element, one side of the metering adjustment element is connected to one side of the observation element; and
the metering adjustment element further includes a knob, a left end surface of the knob is connected to a right side of the pen holder.

Preferably, a right end surface of the knob is provided with a button, a left end surface of the button extends through the knob to inside of the driving rod, one side of the button is slidingly connected to an inner wall of the knob, the side of the button is in sealing contact with an inner wall of the knob, so that the button can be pressed directly to deliver the medicine.

Preferably, a surface of the driving rod is provided with a restoration spring, a left end surface of the button is in sealing and extrusion contact with an inner wall of the driving rod, so that the restoration spring allows the button to be reciprocated in time after pressing the button.

Preferably, an inner wall of a right end of the pen holder is provided with a locking ring, one side of the driving rod is slidingly connected to the locking ring, a right end surface of the restoration spring is connected to an inner wall of the locking ring, so the locking ring can fix the position of the knob.

Preferably, a left end surface of the driving rod is provided with a power spring, a left end surface of the power spring is connected to a right end surface of the ratchet socket, a scale socket is provided with inside the pen holder, a right end surface of the power spring is connected to an inner wall of the scale socket, one side of the scale socket is slidingly connected to an inner wall of the pen holder, so that the scale socket allows the operator to observe metering of output.

Preferably, an inner wall of the pen cap is provided with a medicine cassette, an inner wall of the medicine cassette is provided with a cartridge, an inner wall of the medicine cassette is slidingly connected to a surface of the cartridge, in which the medicines is placed inside the medicine cassette.

Preferably, an inner wall of a right end of the cartridge is provided with a cartridge rubber stopper, a right side of the cartridge rubber stopper is provided with a pushing rod gasket, a left end surface of the pushing rod is extrusion connect with a right side of the cartridge rubber stopper, so that the pushing rod gasket allows the pushing rod to execute lateral linear motion.

Preferably, a top surface of the pen cap is provided with a socket shelf to place the pen cap.

To compare with the prior art, the beneficial effects of the present invention are: the drug delivery device is provided with a metering adjustment mechanism, and is provided with the knob and the scale socket to operate together, so that the drug delivery device can reciprocate when adjusting the dosage to be delivered, so that the metering adjustment is more accurate, and avoids the drug waste. In addition, the button does not shift during the metering adjustment process, so the injection is more convenient, the transmission mechanism of the product is simplified, and the elements is to be optimized.

Specifically, the present invention is provided with the driving ratchet, the ratchet socket and the pushing rod to operate together, so that the force on the cartridge rubber stopper is linear and will not be influenced by external forces in other directions, so that the cartridge rubber stopper can maintain a balanced state to induce the error of the injection dosage. Moreover, by providing a drug delivery mechanism, non-rotating linear motion reduces the decomposition of force and reduces the resistance generated by friction compared with the traditional rotating linear motion. In the case of only linear motion, the pushing rod and the pushing rod gasket and the cartridge rubber stopper will not produce relative displacement or rotation, so the inaccurate injection metering caused by deformation or offset during the extrusion process can be avoided.

### BRIEF DESCRIPTION OF THE DRAWINGS

Fig.1 is a schematic three-dimensional view of the present invention.
Fig. 2 is a cross-sectional view of the present invention.
Fig. 3 is an enlarged schematic diagram of area A in Fig. 2.
Fig. 4 is an enlarged schematic diagram of area B in Fig. 2.
Fig. 5 is an enlarged schematic diagram of area C in Fig. 2.
Fig. 6 is a schematic cross-sectional view of another operating state of the present invention.
In the picture: 1 pen cap, 2 medicine cassette, 3 cartridge, 4 cartridge rubber stopper, 5 power spring, 6 driving rod, 7 restoration spring, 8 button, 9 pushing rod gasket, 10 driving ratchet, 11, ratchet socket, 12 pushing rod, 13 scale socket, 14 pen holder, 15 locking ring, 16 knob, 17 socket shelf, 101 drug administration mechanism, 201 metering adjustment mechanism

### DETAILED DESCRIPTION OF THE PREFERRED EMBODIMENT

The technical solutions in the embodiments of the present invention will be clearly and completely described below with reference to the accompanying drawings in the embodiments of the present invention. Obviously, the described embodiments are only some of the embodiments of the present invention, not all of them. Based on the embodiments of the present invention, all other embodiments obtained by those of ordinary skill in the art without creative efforts fall within the scope of protection of the present invention.

### Embodiment 1

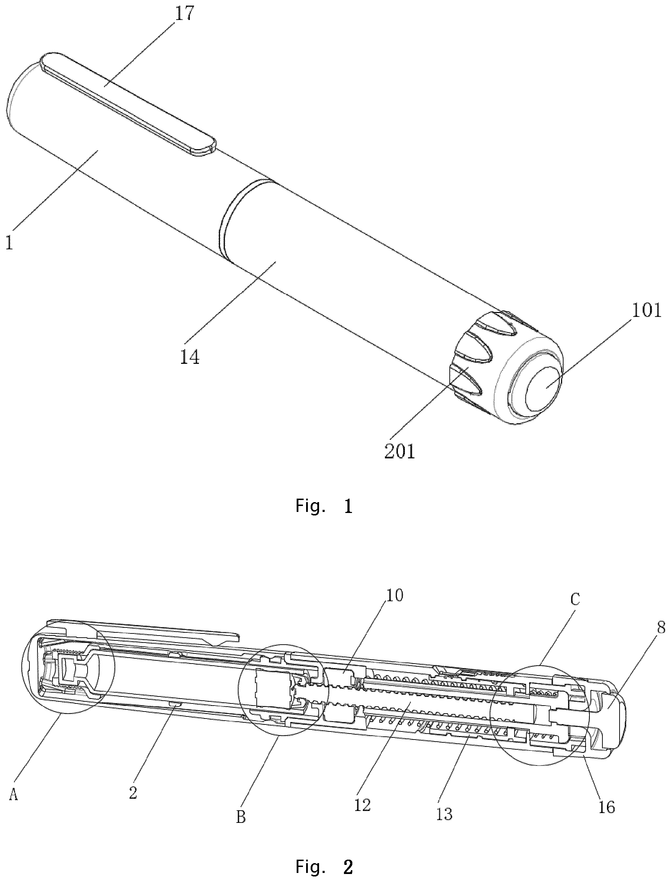

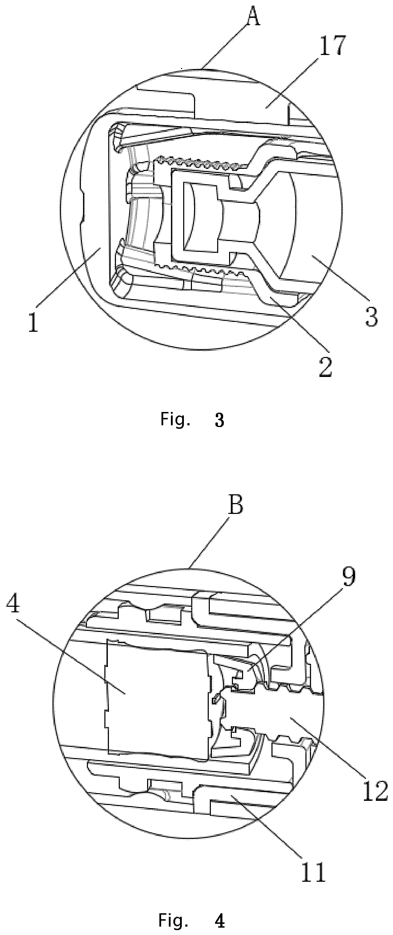

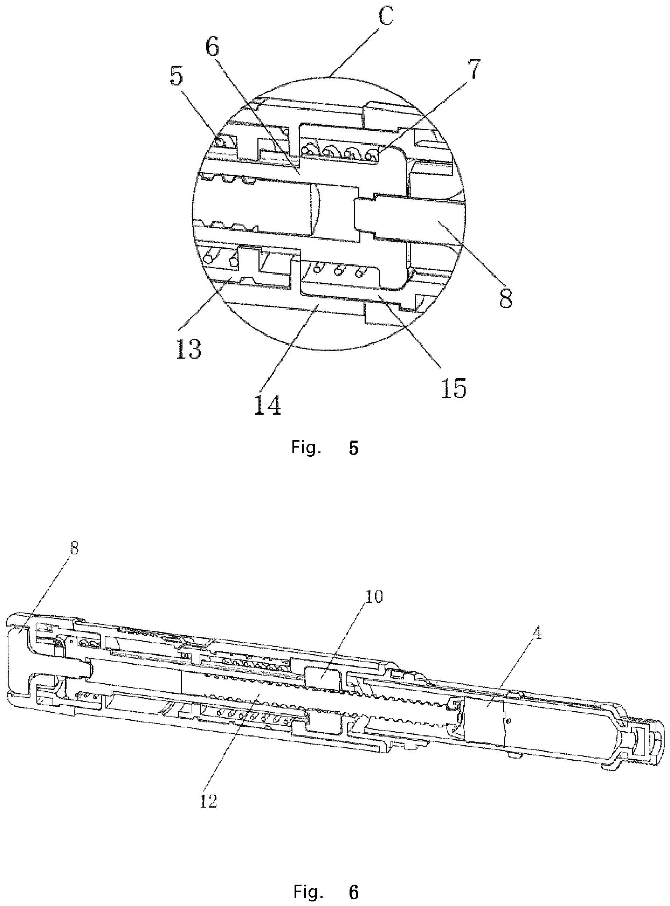

Please refer to Fig. 1 to Fig. 6, the present invention provides a technical solution: a drug delivery device includes a pen cap 1, a right side of the pen cap 1 is provided with a drug administration mechanism 101, a right end surface of the drug administration mechanism 101 is provided with a metering adjustment mechanism 201; the drug administration mechanism 101 further includes a drug administration element and a connection element, in which one side of the drug administration element is connected to one side of the connection element; the connection element further includes pen holder 14, a right side of the pen holder is connected through a right side of the pen cap 1.

A right end surface of a knob 16 is provided with a button 8, a left end surface of the button 8 is extend through the knob 16 to inside of the driving rod 6, one side of the button 8 is slidingly connected to an inner wall of the knob 16, one side of the button 8 is in sealing contact with an inner wall of the knob 16, a driving rod 6 is provided inside the pen holder 14, a surface of the driving rod 6 is covered with a restoration spring 7, a left end surface of the button 8 is in sealing and extrusion contact with an inner wall of the driving rod 6.

A left end surface of the driving rod 6 is covered with a power spring 5, a left end surface of the power spring 5 is connected to a right end surface of the ratchet socket 11, a scale socket 13 is provided inside the pen holder 14, a right end surface of the power spring 5 is connected to an inner wall of the scale socket 13, and one side of the scale socket 14 is slidingly connected to an inner wall of the pen holder 14.

Furthermore, through the operation provided with the drug administration mechanism 101, the non-rotating linear motion reduces the decomposition of force and reduces the resistance generated by friction compared with the traditional rotating linear motion. When only linear motion is generated, the pushing rod 12 and the pushing rod gasket 9 and the cartridge rubber stopper 4 will not generate relative displacement and the rotation at the same time, so the inaccurate injection dosage caused by deformation or offset during the extrusion process can be avoided.

### Embodiment 2

Please continue to refer Fig. 1 to Fig. 6. On the basis of embodiment 1: the metering adjustment mechanism 201 further includes an adjustment element and an observation element, one side of the adjustment element is connected to one side of the observation element; the adjustment element further includes a knob 16, a left end surface of the knob 16 is connected to a right side of the pen holder 14, a pushing rod 12 is provided inside the driving rod 6, one side of the pushing rod 12 is slidingly connected to an inner wall of the driving rod 6.

An inner wall of the pen cap 1 is provided with a medicine cassette 2, an inner wall of the medicine cassette 2 is provided with a cartridge 3, an inner wall of the medicine cassette 2 is slidingly connected to a surface of the cartridge 3, an inner wall of a right end of the cartridge 3 is provided with a cartridge rubber stopper 4, a right side of the cartridge rubber stopper 4 is provided with a pushing rod gasket 9, and a left end surface of the pushing rod 12 is in extrusion contact with a right side of the cartridge rubber stopper 4.

An inner wall of a right end of the pen cap 1 is provided with a ratchet socket 11, a left side of the pen holder 14 is connected through to a right side of the pen cap 1 through the ratchet socket 11, an inner wall of the ratchet socket 11 is provided with a driving ratchet 10, one side of the pushing rod 12 is connected to one side of the driving ratchet 10, and a top surface of the pen cap 1 is provided with a socket shelf 17. An inner wall of a right end of the pen holder 14 is provided with a locking ring 15, one side of the driving rod 6 is slidingly connected to an inner wall of the locking ring 15, and the right end surface of the restoration spring 7 is connected to an inner wall of the locking ring 15.

The medicine in the cartridge 3 can be injected at one time or in multiple batches. It will be discarded after the medicine is injected, and there will not be replaced another new cartridge 3. After the injection is completed, if it has next injection that is to be performed, the operator only needs to remove the used needle and replace with a new needle when the next injection is required.

Furthermore, the drug delivery device of the present invention is provided with a metering adjustment mechanism 201, and by the cooperation of the knob 16 and the scale socket 13, it is possible to reciprocate when adjusting the dosage to be delivered, so the adjustment of the dosage is more accurate to avoid wasting the drug. The button is not moved during the metering adjustment process, so that the injection is more convenient, the transmission mechanism of the product is simplified, and the element(s) is to be optimized.

Moreover, through the cooperation of the driving ratchet 10 and the ratchet socket 11, the pushing rod 12 will only produce linear motion, and will not generate rotational motion, so that the force of the cartridge rubber stopper 4 is linear and will not be influenced by external force in other directions, it effectively improves the defect of the pushing rod in the prior art that has linear motion plus rotational motion, so the cartridge rubber stopper 4 can maintain a balanced state to avoid excessive errors in the injection dosage.

When in use, the user can rotate the knob 16 clockwise to drive the driving rod 6 to rotate, so the scale socket 13 rotates to the right to compress the power spring 5. After adjusting to the required dosage, the knob 16 is loosen, the locking ring 15 is used to restrict the knob 16 from moving.

When the medicine dosage is required to reduce, the knob 16 is rotated count-clockwise to rotate the driving rod 6 to rotate the scale socket 13 to the left, so the power spring 5 is rebounded. It should be noted that the power spring 5 can be a compression spring, a tension spring or a torsion spring. The skilled in the art can select the most appropriate spring type according to actual design requirements and are not limited to the spring type of the present invention.

After adjusting to the required dosage, the knob 16 is loosened, and the position of the knob 16 is restricted by the locking ring 15 to adjust the dosage to the required precise amount. The button 8 is pressed to disengage the driving rod 6 break away from the control of the knob 16, so the restoration spring 7 is compressed, the rod pin of the driving rod 6 enters inside of the driving ratchet 10, the compressed power spring 5 is bounded to drive the scale socket to rotate counter-clockwise, so the driving rod 6 drives the driving ratchet 10 to move the pushing rod 6 to the left. Thereby, the cartridge rubber stopper 4 is extruded by the pushing rod gasket 9 to perform drug administration.

Although the embodiments of the present invention have been shown and described, the ordinary skill in the art will appreciate that various changes, modifications, substitutions and changes can be made to the embodiments without departing from the principles and spirit of the invention. Alternatively, the scope of the invention is defined by the appended claims and their equivalents.

## Claims

1. A drug delivery device, the **characterized in that**, the drug delivery device comprising:
a pen cap (1), a right side of the pen cap (1) is fixedly provided with a drug administration mechanism (101), a right end surface of the drug delivery mechanism (101) is fixedly provided with a metering adjustment mechanism (201), an inner wall of a right end surface of the pen cap (1) is provided with a ratchet socket (11), an inner wall of the ratchet socket (11) is provided with a driving ratchet (10);
the drug administration mechanism (101) further includes a drug administration element and a connection element, one side of the drug administration element is connected to one side of the connection element;
the connection element further includes a pen holder (14), a left side of the pen holder (14) and a right side of the pen cap (1) are connected through the ratchet socket (11), a driving rod (6) is provided inside the pen holder (14), a pushing rod (12) is provided inside the driving rod (6), one side of the pushing rod (12) is slidingly connected to an inner wall of the driving rod (6), and one side of the pushing rod (12) is connected to one side of the driving ratchet (10);
the metering adjustment mechanism (201) further includes an adjustment element and an observation element, one side of the adjustment element is connected to one side of the observation element; and
the adjustment element further includes a knob (16), and a left end surface of the knob (16) is connected to a right side of the pen holder (14).

2. The drug delivery device according to claim **1,** wherein a right end surface of the knob (16) is provided with a button (8), a left end surface of the button (8) extends through the knob (16) to the inside of the driving rod (6), one side of the button (8) is slidingly connected to an inner wall of the knob (16), and one side of the button (8) is in sealing contact with an inner wall of the knob (16).

3. The drug delivery device according to claim 2, wherein a surface of the driving rod (6) is fixed with a restoration spring (7), and a left end surface of the button (8) is in sealing and extrusion contact with an inner wall of the driving rod (6).

4. The drug delivery device according to claim 3, wherein a locking ring (15) is provided on an inner wall of a right end of the pen holder (14), one side of the driving rod (6) is slidingly connected to an inner wall of the locking ring (15), and a right end surface of the reciprocated spring (7) is connected to an inner wall of the locking ring (15).

5. The drug delivery device according to claim 1, wherein a left end surface of the driving rod (6) is provided with a power spring (5), a left end surface of the power spring (5) is connected to a right end surface of the ratchet socket (11), a scale socket (13) is provided with inside the pen holder (14), a right end surface of the power spring (5) is connected to an inner wall of the scale socket (13), and one side of the scale socket (13) is slidingly connected to an inner wall of the pen holder (14).

6. The drug delivery device according to claim **1,** wherein an inner wall of the pen cap (1) is fixedly provided with a medicine cassette (2), an inner wall of the medicine cassette (2) is provided with a cartridge (3), and an inner wall of the medicine cassette (2) is slidingly connected to a surface of the cartridge (3).

7. The drug delivery device according to claim 6, wherein an inner wall of a right end of the cartridge (3) is provided with a cartridge rubber stopper (4), a right side of the cartridge rubber stopper (4) is provided with a pushing rod gasket (9), a left end surface of the pushing rod (12) is in extrusion contact with a right side of the cartridge rubber stopper (4).

8. The drug delivery device according to claim 1, wherein a top surface of the pen cap (1) is provided with a socket shelf (17).
